# EUROPEAN PATENT APPLICATION

(11) **EP 1 681 009 A1**
(43) Date of publication of application: **19.07.2006**
(21) Application number: 04792661.3
(22) Date of filing: 20.10.2004
(51) Int. Cl.: A61B 1/00

(54) **IMAGE PROCESSING DEVICE, IMAGE PROCESSING METHOD, AND IMAGE PROCESSING PROGRAM**

(30) Priority: 27.10.2003 JP 2003365636; 04.11.2003 JP 2003373927
(71) Applicant: Olympus Corporation, Tokyo 151-0072 (JP)
(72) Inventor: NAKAMURA, Kenji, Chiba-shi, Chiba 2610013 (JP); HIRAKAWA, Katsumi, Sagamihara-shi, Kanagawa 2291103 (JP)
(74) Representative: von Hellfeld, Axel
(86) International application number: PCT/JP2004/015495
(87) International publication number: WO 2005/039399

(57) **Abstract**

It is a solution of the problems to provide an image processing apparatus (7) that obtains a plurality of images taken by a capsule endoscope (1), determines whether the image is obtained by photographing an inside of a body or not, extracts the image determined as the image obtained by photographing the inside of the body, determines whether the extracted images are substantially identical to each other or not, extracts the images determined as different images, and then extracts the images in which an affection region is taken.

## Description

### TECHNICAL FIELD

The present invention relates to image processing of an enormous amount of images taken by a medical instrument, particularly by a capsule endoscope.

### BACKGROUND ART

Recently, a swallowable capsule endoscope has entered the field of endoscope. The capsule endoscope is provided with an image pickup function and a wireless communication function. After the capsule endoscope is swallowed through a mouth of a patient for the purpose of observation (examination), the capsule endoscope has the function of sequentially taking the images of organs such as a gaster and a small intestine for an observation period until the capsule endoscope is naturally discharged from a human body (Patent Document 1).

The image data, which is taken by the capsule endoscope in the body during the observation period, is sequentially transmitted to the outside by wireless communication and stored in a memory. For the observation period until the capsule endoscope is discharged after the patient swallows the capsule endoscope, the patient can freely go about because the patient carries a receiver including the wireless communication function and a memory function. After the observation, a doctor or a nurse can make a diagnosis by displaying the organ image based on the image data stored in the memory.

Recently, M2A (registered trademark) of Given Imaging Ltd. in Israel and NORIKA (registered trademark) of RF SYSTEM lab. in Japan can be cited as an example of this type of capsule endoscope, and the capsule endoscopes evolve into a practical application stage.

Patent Document 1: United States Patent Application Publication No. 2002/0093484

### DISCLOSURE OF INVENTION

### PROBLEM TO BE SOLVED BY THE INVENTION

However, in the conventional capsule endoscope, since the capsule endoscope takes the images of organs for the observation period until the capsule endoscope is naturally discharged after the subject swallows the capsule endoscope, an observation (examination) time is prolonged for many hours, e.g., eight hours, unlike the normal endoscope. The image is taken, e.g., in each 2 frames per second during the observation time. Therefore, the enormous number of images is taken in time series.

In a diagnosis stage, when the inside image of the test object such as a region and an organ is confirmed, it is necessary to perform searching from the enormous number of images. Therefore, significant amounts of time and labor are consumed.

In view of the foregoing, it is an object of the invention to provide an image processing apparatus, an image processing method, and an image processing program in which the number of images can be decreased as much as possible by extracting and referring to only the region of the observation object in the images taken by the capsule endoscope.

### MEANS FOR SOLVING PROBLEM

According to the invention as set forth in claim 1, it is a solution of the problems to provide an image processing apparatus that performs image processing of a plurality of images taken by a medical instrument, and includes an intracorporeal image determination unit that determines whether the image is an intracorporeal image obtained by photographing an inside of a body or not; and an intracorporeal image extraction unit that extracts the intracorporeal image based on a determination result by the intracorporeal image determination unit.

According to the invention as set forth in claim 2, it is a solution of the problems to provide the image processing apparatus according to claim 1 wherein the intracorporeal image determination unit determines whether the image is the intracorporeal image based on color component information on a pixel value included in the image.

According to the invention as set forth in claim 3, it is a solution of the problems to provide the image processing apparatus according to claim 1 wherein the observation-object image determination unit compares a predetermined threshold to the color component information on the pixel value included in each image.

According to the invention as set forth in claim 4, it is a solution of the problems to provide the image processing apparatus according to claim 3 wherein the threshold is based on the color component information on the pixel value of the observation object in the observation-object image.

According to the invention as set forth in claim 5, it is a solution of the problems to provide the image processing apparatus according to claim 2 wherein the color component information is indicated by at least one of color components of tint elements x, y of an XYZ colorimetric system, tint elements u, v of a CIE U*V*W* color space, tint elements u', v' of a CIE LUV color space, tint elements a*, b* of a CIE LAB color space, and a ratio to an RGB signal value.

According to the invention as set forth in claim 6, it is a solution of the problems to provide the image processing apparatus according to claim 1 wherein the intracorporeal image determination unit determines that all the undetermined images are the intracorporeal images when the intracorporeal image determination unit determines that a predetermined number of images among the plurality of images taken by the medical instrument are the intracorporeal images.

According to the invention as set forth in claim 7, it is a solution of the problems to provide the image processing apparatus according to claim 1 which further includes an image identical determination unit that determines whether two given images are substantially identical to each other or different from each other among the intracorporeal images extracted by the intracorporeal image extraction unit; and a different image extraction unit that extracts the different image based on a determination result by the image identical determination unit.

According to the invention as set forth in claim 8, it is a solution of the problems to provide the image processing apparatus according to claim 7 wherein the image identical determination unit computes a difference between pixel values of two continuous intracorporeal images, and determines whether the two given images are substantially identical to each other or different from each other based on the difference.

According to the invention as set forth in claim 9, it is a solution of the problems to provide the image processing apparatus according to claim 1 which further includes an intracorporeal image determination unit that determines whether the image is an intracorporeal image obtained by photographing an inside of a body or not; and an intracorporeal image extraction unit that extracts the intracorporeal image based on a determination result by the intracorporeal image determination unit.

According to the invention as set forth in claim 10, it is a solution of the problems to provide the image processing apparatus according to claim 1 wherein the medical instrument is a capsule endoscope.

According to the invention as set forth in claim 11, it is a solution of the problems to provide an image processing program which causes a computer to perform image processing of a plurality of images taken by a medical instrument, and causes the computer to perform an intracorporeal image determination step that determines whether the image is an intracorporeal image obtained by photographing an inside of a body or not; and an intracorporeal image extraction step that extracts the intracorporeal image based on a determination result by the intracorporeal image determination step.

According to the invention as set forth in claim 12, it is a solution of the problems to provide the image processing program according to claim 11 which causes the computer to perform, in the intracorporeal image determination step, determining whether the image is the intracorporeal image based on color component information on a pixel value included in the image.

According to the invention as set forth in claim 13, it is a solution of the problems to provide the image processing program according to claim 11 which causes the computer to perform, in the observation-object image determination step, comparing a predetermined threshold to the color component information on the pixel value included in each image.

According to the invention as set forth in claim 14, it is a solution of the problems to provide the image processing program according to claim 13 wherein the threshold is based on the color component information on the pixel value of the observation object in the observation-object image.

According to the invention as set forth in claim 15, it is a solution of the problems to provide the image processing program according to claim 12 wherein the color component information is indicated by at least one of color components of tint elements x, y of an XYZ colorimetric system, tint elements u, v of a CIE U*V*W* color space, tint elements u', v' of a CIE LUV color space, tint elements a*, b* of a CIE LAB color space, and a ratio to an RGB signal value.

According to the invention as set forth in claim 16, it is a solution of the problems to provide the image processing program according to claim 11 which causes the computer to perform, in the intracorporeal image determination step, determining that all the undetermined images are the intracorporeal images when it is determined that a predetermined number of images among the plurality of images taken by the medical instrument are the intracorporeal images.

According to the invention as set forth in claim 17, it is a solution of the problems to provide the image processing program according to claim 11 which further causes the computer to perform an image identical determination step that determines whether two given images are substantially identical to each other or different from each other among the intracorporeal images extracted by the intracorporeal image extraction step; and a different image extraction step that extracts the different image based on a determination result by the image identical determination step.

According to the invention as set forth in claim 18, it is a solution of the problems to provide the image processing program according to claim 17 which causes the computer to perform, in the image identical determination step, computing a difference between pixel values of two continuous intracorporeal images; and determining whether the two given images are substantially identical to each other or different from each other based on the difference.

According to the invention as set forth in claim 19, it is a solution of the problems to provide the image processing program according to claim 11 which further causes the computer to perform an intracorporeal image determination step that determines whether the image is an intracorporeal image obtained by photographing an inside of a body or not; and an intracorporeal image extraction step that extracts the intracorporeal image based on a determination result by the intracorporeal image determination step.

According to the invention as set forth in claim 20, it is a solution of the problems to provide the image processing program according to claim 11 wherein the medical instrument is a capsule endoscope.

According to the invention as set forth in claim 21, it is a solution of the problems to provide an image processing method which performs image processing of a plurality of images taken by a medical instrument, and includes an intracorporeal image determination step that determines whether the image is an intracorporeal image obtained by photographing an inside of a body or not; and an intracorporeal image extraction step that extracts the intracorporeal image based on a determination result by the intracorporeal image determination step.

According to the invention as set forth in claim 22, it is a solution of the problems to provide the image processing method according to claim 21 wherein the intracorporeal image determination step includes determining whether the image is the intracorporeal image based on color component information on a pixel value included in the image.

According to the invention as set forth in claim 23, it is a solution of the problems to provide the image processing method according to claim 21 wherein the observation-object image determination step includes comparing a predetermined threshold to the color component information on the pixel value included in each image.

According to the invention as set forth in claim 24, it is a solution of the problems to provide the image processing method according to claim 23 wherein the threshold is based on the color component information on the pixel value of the observation object in the observation-object image.

According to the invention as set forth in claim 25, it is a solution of the problems to provide the image processing method according to claim 22 wherein the color component information is indicated by at least one of color components of tint elements x, y of an XYZ colorimetric system, tint elements u, v of a CIE U*V*W* color space, tint elements u', v' of a CIE LUV color space, tint elements a*, b* of a CIE LAB color space, and a ratio to an RGB signal value.

According to the invention as set forth in claim 26, it is a solution of the problems to provide the image processing method according to claim 21 wherein the intracorporeal image determination step includes determining that all the undetermined images are the intracorporeal images when it is determined that a predetermined number of images among the plurality of images taken by the medical instrument are the intracorporeal images.

According to the invention as set forth in claim 27, it is a solution of the problems to provide the image processing method according to claim 21. which further includes an image identical determination step that determines whether two given images are substantially identical to each other or different from each other among the intracorporeal images extracted by the intracorporeal image extraction step; and a different image extraction step that extracts the different image based on a determination result by the image identical determination step.

According to the invention as set forth in claim 28, it is a solution of the problems to provide the image processing method according to claim 27 wherein the image identical determination step includes computing a difference between pixel values of two continuous intracorporeal images, and determining whether the two given images are substantially identical to each other or different from each other based on the difference.

According to the invention as set forth in claim 29, it is a solution of the problems to provide the image processing method according to claim 21 which further includes an intracorporeal image determination step that determines whether the image is an intracorporeal image obtained by photographing an inside of a body or not; and an intracorporeal image extraction step that extracts the intracorporeal image based on a determination result by the intracorporeal image determination step.

According to the invention as set forth in claim 30, it is a solution of the problems to provide the image processing method according to claim 21 wherein the medical instrument is a capsule endoscope.

The unnecessary image can be removed to extract only the necessary image by the configurations of the invention as set forth in claims 1, 11, and 21.

Only the image having specific tint of the body-cavity image can be extracted by the configurations of the invention as set forth in claims 2, 12, and 22.

Only the image having the specific tint of the region or organ of the observation object can be extracted by the configurations of the invention as set forth in claims 3, 13, and 23.

Only the image having the specific tint of the region or organ of the observation object can be extracted by the configurations of the invention as set forth in claims 4, 14, and 24.

The tint of various colorimetric systems or color spaces can be used by the configurations of the invention as set forth in claims 5, 15, and 25.

The speed enhancement of the processing can be achieved by the configurations of the invention as set forth in claims 6, 16, and 26.

The unnecessary image can be removed to extract only the necessary image by the configurations of the invention as set forth in claims 7, 17, and 27.

Whether two continuous frames are substantially the same image or different images can be determined by the configurations of the invention as set forth in claims 8, 18, and 28.

The unnecessary image can be removed to extract only the necessary image by the configurations of the invention as set forth in claims 9, 19, and 29.

Only the necessary image can efficiently be obtained from the enormous amount of images, taken by the capsule endoscope, by the configurations of the invention as set forth in claims 10, 20, and 30.

### EFFECT OF THE INVENTION

According to the present invention, an efficient medical practice and a shorter examination can be achieved by cutting down (extremely rapidly) the number of images which should be watched in the diagnosis by the doctor.

### BRIEF DESCRIPTION OF DRAWINGS

FIG. 1 is a diagram showing a capsule endoscope and peripherals used in a body cavity test in an embodiment;
FIG. 2 is a diagram showing an internal configuration of a workstation 7 which performs image processing of image data taken by the capsule endoscope in the embodiment;
FIG. 3 is a diagram showing a whole flow of the image processing in the embodiment;
FIG. 4 is a diagram showing a detailed processing flow of an extracorporeal and intracorporeal discrimination process of an image in S1 in FIG. 3;
FIG. 5 is a diagram showing a detailed processing flow of a different and identical discrimination process of the image in S2 in FIG. 3;
FIG. 6 is a diagram showing a detailed processing flow of a necessary and unnecessary discrimination process of the image in S3 in FIG. 3;
FIG. 7 is a diagram (example 1) showing a whole flow of image processing in a second embodiment; and
FIG. 8 is a diagram (example 2) showing a whole flow of the image processing in the second embodiment.

### EXPLANATIONS OF LETTERS OR NUMERALS

1 capsule endoscope
2 patient
3 jacket
4 antenna unit
5 external device
6 cradle
7 workstation
8 display
9 keyboard
10 mouse
11 antenna
21 image determination unit
21a intracorporeal image determination unit
21b observation-object image determination unit
21c image identical determination unit
22 image extraction unit
22a intracorporeal image extraction unit
22b observation-object image extraction unit
22c different image extraction unit
23 input I/F
24 output I/F
25 storage unit
26 control unit

### BEST MODE(S) FOR CARRYING OUT THE INVENTION

First Embodiment
Image processing in which only the image (hereinafter referred to as necessary image) of the region, which is of the test object (observation object), is extracted from enormous amounts of image data to set at display object will be described in a first embodiment. The necessary image and unnecessary image data (extracorporeal images and images other than the region which is of the test object (observation object)) will be first described below.

Since photographing by the capsule endoscope is usually started immediately before the capsule endoscope is swallowed, the extracorporeal image and the image data such as an intraoral image which is not the photographing object, i.e., the unnecessary image data is included.

Since the capsule endoscope travels in the body cavity by peristaltic motion of the alimentary system organ, sometimes there is a possibility that traveling of the capsule endoscope is temporarily stopped, when a short break of the peristaltic motion is generated or when the movement of the capsule endoscope is suppressed by body cavity conditions (caused by an affection, an alimentary port, or the like). However, even in this case, since the photographing is continually performed, the images taken in the short break of the peristaltic motion are equal to one another or substantially equal to one another.

For example, in the case where only the image of the gaster is necessary, the images of other organs such as an esophagus and the small intestine are the unnecessary images. In the case where only the image of the affection region is necessary, the images in which regions other than the affection region are taken are unnecessary.

Thus, in the enormous mounts of taken image data, since the images except for the observation object image have little need for confirmation in the diagnosis, only the necessary image is extracted. The first embodiment will be now described in detail below.

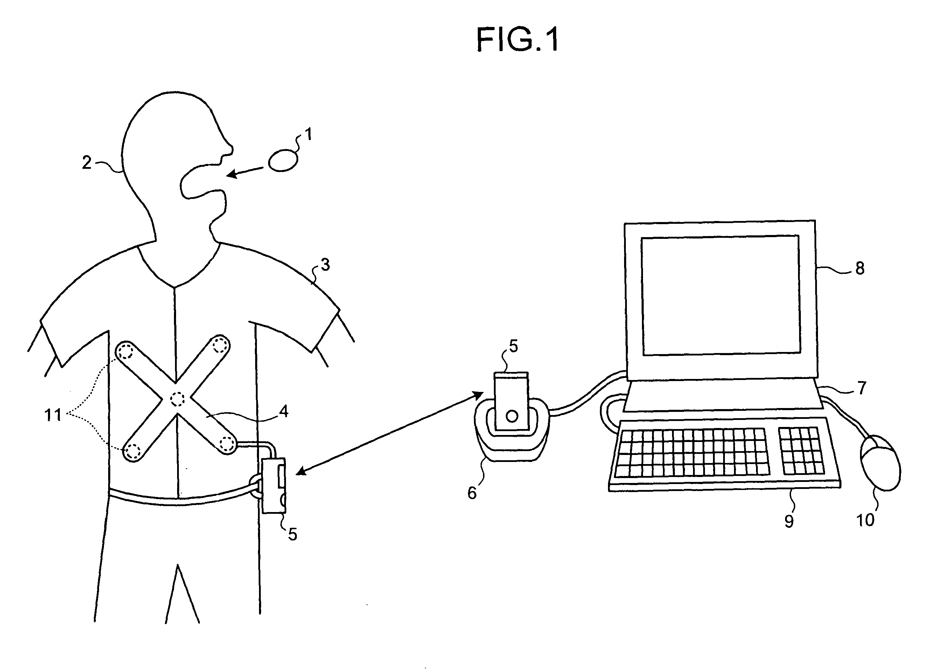

FIG. 1 is a diagram showing a capsule endoscope and peripherals used in a body cavity test in the first embodiment. As shown in FIG. 1, a test system in which a capsule endoscope 1 is used includes the capsule endoscope 1 which is swallowed through a mouth of a patient 2 to examine the body cavity, and an external device 5 which is arranged outside the body of the patient 2 and serves as a receiving device connected to an antenna unit 4 receiving image data taken by the capsule endoscope 1 through wireless communication.

A workstation 7 (workstation 7 is used in the first embodiment) such as a personal computer or a workstation is configured to capture image information through a portable storage medium such as CompactFlash (registered trademark) memory. In testing the body cavity, the portable storage medium is mounted on the external device 5 to record the image information which is transmitted from the capsule endoscope 1 and received by the external device 5. The workstation 7 functions as an image processing apparatus to extract images necessary to the diagnosis from enormous amount of images.

Shown in FIG. 1, the external device 5 can be electrically connected to the workstation (image processing apparatus) 7 by mounting the external device 5 on a cradle 6 or through a USB cable (not shown) and the like. Therefore, the workstation 7 can capture the image data stored in the portable storage medium inserted into the external device 5. Alternatively, the image data stored in the portable storage medium may be read and captured into the workstation 7 by connecting a reading device as the portable storage medium to the workstation 7 to insert the portable storage medium into the reading device.

The capture of the images is performed by an operation of a console device such as a keyboard 9 or a mouse 10. The images captured in the workstation 7 can be displayed on a display 8 or outputted to a printer.

As shown in FIG. 1, the antenna unit 4, to which plural antennas 11 are attached, is mounted to a jacket 3 which the patient 2 wears. When the endoscope test is performed by swallowing the capsule endoscope 1, the image data taken by the capsule endoscope 1 is transmitted to the antennas 11 through wireless communication and thus is received by the antenna unit 4. The image data is stored in the external device 5 connected to the antenna unit 4. The external device 5 is attached to, e.g., a belt of the patient 2 with a detachable hook.

The capsule endoscope 1 is formed in a capsule shape with a water-proof structure and includes an image pickup unit which takes pictures of the body cavity, an illumination unit which illuminates the photographing object, a transmission unit which transmits the taken image to the antenna 11, a battery which drives the image pickup unit, the illumination unit, and the transmission unit, and a power supply board unit.

For starting up the capsule endoscope 1, an ON/OFF switch, which serves as electric power supply start means, is provided in the capsule, and turning on the switch starts the electric power supply for the image pickup unit, the illumination unit, and the other units. The ON/OFF switch is provided in the power supply board unit of the capsule endoscope 1 and is a switch which starts the electric power supply to each unit of the capsule endoscope 1 from the battery (for example, silver oxide cell) provided in the power supply board unit.

An external magnet which generates magnetic power from the outside (for example, the external magnet is provided in a package packing the capsule endoscope 1) of the capsule endoscope 1 biases the ON/OFF switch to an OFF state. An internal magnet is provided near the ON/OFF switch in the capsule endoscope 1 and biases the ON/OFF switch to an ON state.

Therefore, the ON/OFF switch can be changed from an OFF position to an ON position by keeping the capsule endoscope 1 away from the external magnet, in other words, by taking out the capsule endoscope 1 from a package packing the capsule endoscope 1, which starts up the capsule endoscope 1 to start the photographing.

Accordingly, since the photographing is started by taking out the capsule endoscope 1 from the package packing the capsule endoscope 1, the extracorporeal images unnecessary to the diagnosis are taken before the capsule endoscope 1 is taken into the body.

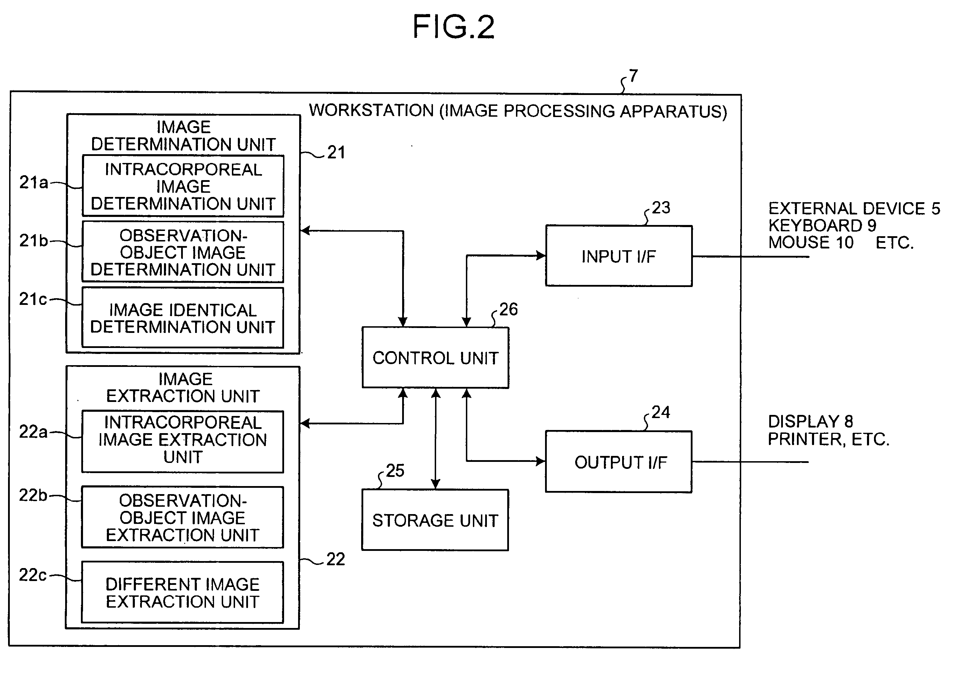

A configuration of the workstation 7 which functions as an example of the image processing apparatus in claims will be described below. FIG. 2 is a schematic diagram of an internal configuration of the workstation 7 which performs the image processing of image data taken by the capsule endoscope 1 in the first embodiment. The workstation 7 includes an image determination unit 21 which performs a determination process on a large amount of inputted images based on a predetermined criterion, an image extraction unit 22 which extracts a predetermined image from the large amount of images based on the result of the determination process in the image determination unit 21, an input I/F 23 which accepts predetermined data such as the image from the external device 5, an output I/F 24 which outputs the image extracted by the image extraction unit 22 to the display 8 or the like, a storage unit 25 which stores data such as the image to be processed, and a control unit 26 which controls operations of the image determination unit 21 and the like.

The image determination unit 21 determines whether each of the many images inputted from the external device 5 satisfies the predetermined criterion or not. Specifically the image determination unit 21 includes an intracorporeal image determination unit 21a, an observation-object image determination unit 21b, and an image identical determination unit 21c, which each perform determination processes based on different criteria. The intracorporeal image determination unit 21a functions in a later-described image determination in an intracorporeal and extracorporeal discrimination process of the image. The observation-object image determination unit 21b functions in a later-described necessary and unnecessary discrimination process of the image. The image identical determination unit 21c functions in a later-described different and identical discrimination process of the image.

The image extraction unit 22 extracts the predetermined image based on the determination result in the image determination unit 21. Specifically the image extraction unit 22 includes an intracorporeal image extraction unit 22a, an observation-object image extraction unit 22b, and a different image extraction unit 22c, which each perform image extraction processes based on the determination results under different conditions. The intracorporeal image extraction unit 22a is used in reading the image which is determined as the intracorporeal image by the intracorporeal image determination unit 21a. The observation-object image extraction unit 22b is used in reading the image which is determined as the observation object by the observation-object image determination unit 21b. The different image extraction unit 22c is used in reading the image which is determined as the different image by the image identical determination unit 21c. In the first embodiment, the intracorporeal image extraction unit 22a is used in reading the image which is the processing object of the necessary and unnecessary discrimination process performed by the observation-object image determination unit 21b (described later), and the observation-object image extraction unit 22b is used in reading the image which is the processing object of the different and identical discrimination process performed by the image identical determination unit 21c (described later). The different image extraction unit 22c is used in reading the narrowed image after the process performed by the image identical determination unit 21c is ended, and the image read by the different image extraction unit 22c is displayed on the display 8 or the like.

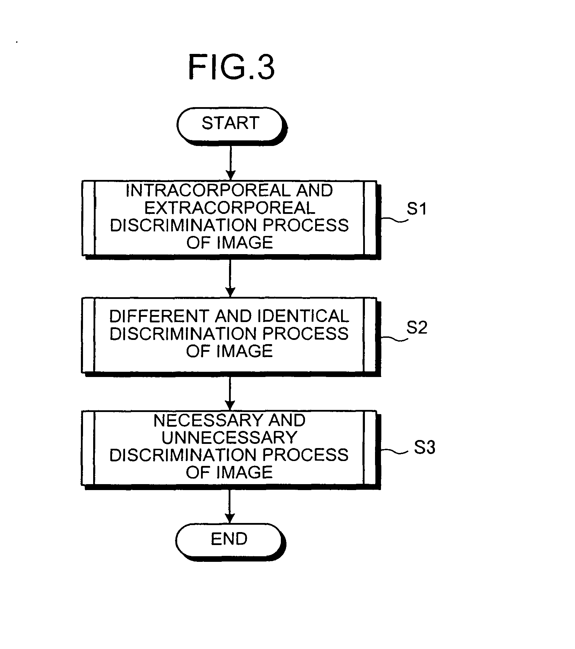

The configuration of the workstation 7 of FIG. 2 is schematically shown by way of example only for the purpose of easy explanation about the image processing apparatus. In the components shown in FIG. 2, for example, actually the image determination unit 21, the image extraction unit 22, and the control unit 26 are usually realized by using a predetermined program in CPU (Central Processing Unit), RAM (Random Access Memory), ROM (Read Only Memory), and the like which are included in the workstation 7. An image processing program which is described such that processes shown in flow from FIG. 3 are executed on CPU (computer) is used as the predetermined program. Needless to say, it is not necessary that the image processing apparatus be interpreted while the image processing apparatus is limited to the above configuration. For example, the image processing apparatus may be realized by an apparatus in which the components shown in FIG. 2 are implemented in a hardware manner.

The image data taken by the capsule endoscope 1 is successively transmitted to the external device 5 and stored in the portable storage medium of the external device 5. The stored image data is, as described above, electrically connected to the workstation 7 by mounting the external device 5 on the cradle 6 or by setting the portable storage medium in the reading device, and the image data is stored in a storage unit 25 of the workstation 7. Thus, the images taken by the capsule endoscope 1 are captured in the workstation 7. The predetermined processes are performed on the image data captured in the workstation 7 through the image processing in the first embodiment, and the image is displayed on the display 8.

FIG. 3 shows a whole flow of the image processing of the image taken by the capsule endoscope 1 in the first embodiment. First a user starts up the image processing apparatus, and the predetermined number of images is inputted as data through the external device 5 and stored in the storage unit 25. Then, the processes according to the flow of FIG. 3, that is, the intracorporeal and extracorporeal discrimination process (Step 1, hereinafter Step is abbreviated to S) of the image, the different and identical discrimination process (S2) of the image, and the necessary and unnecessary discrimination process (S3) of the image are performed on the stored image. As described above, when the image determination unit 21 and the like are realized by using the predetermined program with CPU and the like, the user operates the input device such as the mouse 10 to start up the image processing program previously installed in the storage unit 25 and the like of the workstation 7, and CPU which receives a command for starting up the program reads the installed image processing program to perform the flow of FIG. 3.

In the intracorporeal and extracorporeal discrimination process (S1) of the image, a process of removing the unnecessary extracorporeal images from the data taken by the capsule endoscope 1 to obtain only the intracorporeal images which are of the necessary images is performed. In the different and identical discrimination process (S2) of the image, a process of removing substantially the same images from the intracorporeal images to obtain the different images is performed. In the necessary and unnecessary discrimination process (S3) of the image, a process of obtaining the image data of the observation object is performed.

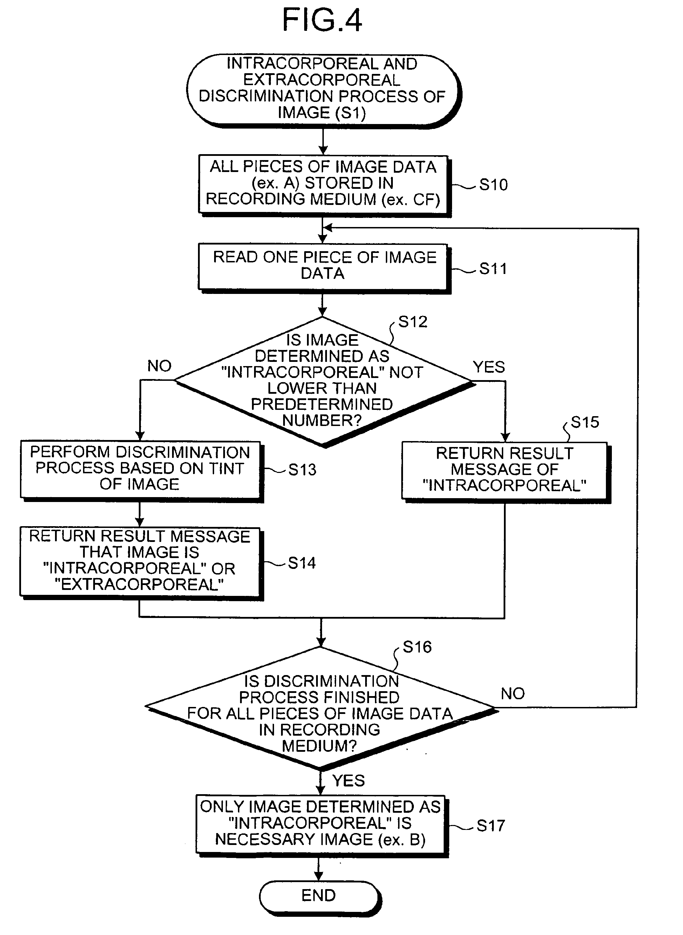

FIG. 4 shows a detailed processing flow of the extracorporeal and intracorporeal discrimination process of the image in S1 of FIG. 3. In the flow of FIG. 4, a discrimination process, in which the pieces of image data stored in the recording medium in the order of photographing are sequentially read, RGB data is converted into XYZ data, and it is determined whether the image is intracorporeal image or the extracorporeal image by a later-described threshold process of an xy chromaticity value, is performed.

The RGB data means image data expressed by an RGB colorimetric system of three primary colors of R (red), G (Green), and B (Blue). The XYZ data means image data expressed by an XYZ colorimetric system. The XYZ colorimetric system is a basic colorimetric system which is defined in order to display a color stimulus specification by International Commission on Illumination (CIE). In the XYZ colorimetric system, even a bright color which cannot be expressed in the RGB colorimetric system can be expressed. Hereinafter a color expressed by XYZ colorimetric system is referred to as tint.

The flow of FIG. 4 will be described below.

It is assumed that the number of images of the image data stored in the storage unit 25 of the workstation 7 after the image data is stored in the recording medium (for example, CompactFlash (registered trademark)) is set at A, and A is assigned to a variable TA for indicating the number of total taken images (S10). Here, only the images to be processed (target folder or the like) may definitely be set at "total pieces of image data A" among the pieces of image data stored in the storage unit 25.

Variable CntA used as a counter is set at 1 (CntA = 1) to read a first piece of image data (S11). It is determined whether the number of images determined as "intracorporeal image" is not lower than a predetermined number (S12). When the flow passes initially through S12, the flow goes to a direction of "No" because the later-described discrimination of "intracorporeal image" is not made yet.

When the flow goes to the direction of "No" in S12, a discrimination process is performed based on the tint of the image (S13). In this process, first RGB data is converted into XYZ data. Since the image data captured in the workstation 7 is RGB data, the image data is converted into XYZ data. The conversion is performed by a general technique, so that the description will be omitted.

The xy chromaticity value is determined from the XYZ data. It is determined whether the xy chromaticity value exists within a predetermined threshold range or not. At this point, the threshold range is set based on a general value distribution of the xy chromaticity values of intracorporeal image data. Therefore, when the computed xy chromaticity value exists within the threshold range, it is interpreted that the image data is data taken in the body. When the computed xy chromaticity value is lower than the threshold range, it is interpreted that the image data is data taken outside the body.

When the xy chromaticity value computed in S13 exists within the threshold range, a message that the image is the "intracorporeal" image is returned. When the xy chromaticity value computed in S13 exists out of the threshold range, a message that the image is the "extracorporeal" image is returned (S14). Then, CntA is incremented (CntA = CntA + 1).

Then, in S16, it is determined whether the processes are finished for the total pieces of obtained image data A or not (S16). Specifically, the flow goes to the direction of "Yes" if TA < CntA, and the flow goes to the direction of "No" if TA ≥ CntA. Since CntA = 2, the flow goes to the direction of "No" (if TA ≠ 1), the second image is read to perform the processes of S11 → S12 → S13 → S14 → S16, CntA is incremented, and then the same processes are performed on the images subsequent to the second image. These processes are repeated.

Then, in S12, when the number of images determined as the intracorporeal images reaches the predetermined number, a result message of "intracorporeal" is returned (S15). Accordingly, the processes of S11 → S12 → S15 → S16 are performed on the images after the number of images determined as the intracorporeal images reaches the predetermined number, and a result message of "intracorporeal" is returned without condition. These processes are based on the fact that only the intracorporeal images are taken after the capsule endoscope 1 existing outside the body is swallowed through the mouth.

Therefore, when the discrimination of the "intracorporeal" image is made from a given frame, it is determined that all the images subsequent to the given frame are the "intracorporeal" image, and the threshold discrimination process in S13 is terminated, so that the speed enhancement of the processing can be achieved.

When the processes are finished for the total pieces of obtained image data A, TA < CntA. Therefore, the flow goes to the direction of "Yes" in S16, and the image in which the result message of "intracorporeal" is returned in S14 or S15 is extracted (S17). Then, the flow is ended. In the first embodiment, the number of all images and the counter are used. However, even if the number of all images and the counter are not used, the process in which "the image files are sequentially read from the first image file, and when the next file is found, the flow goes to the direction of 'No' in S16, otherwise the flow goes to the direction of 'Yes'" may be performed. The number of images extracted in S17 is set at B.

Although the xy chromaticity value is used in the first embodiment, the invention is not limited to the xy chromaticity value. Instead of the xy chromaticity value, any discrimination criterion can be used as long as a factor associated with the tint such as hue and chroma such as L*a*b* or L*u*V* is adopted.

The RGB colorimetric system may be used without converting the captured image into other colorimetric systems or color spaces. In this case, the values of R/G, R/B, and the like (or values of G/R and B/R) may be used as the threshold of the criterion from the RGB signal values.

In S13, when the tint can be determined by any value obtained from the image, the discrimination process is not limited to the kind of RGB or the colorimetric system.

Thus, only the intracorporeal images can be extracted from the pieces of image data, taken by the capsule endoscope 1, by the processes of the flow shown in FIG. 4.

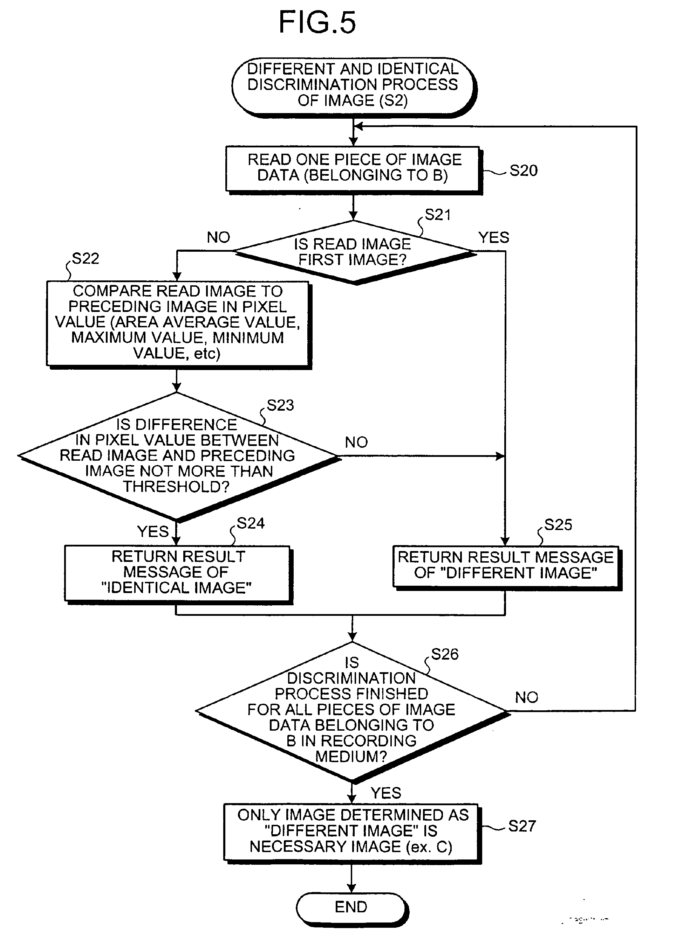

FIG. 5 shows a detailed processing flow of the different and identical discrimination process of the image in S2 of FIG. 3. In the flow of FIG. 5, for example, an average pixel value of the preceding frame and the object frame is examined, and when a change amount of average pixel value is not more than (or lower than) a certain threshold, it is determined that the images are identical to each other, otherwise it is determined that the images are different from each other. Then, the image determined as the different image is extracted. The flow of FIG. 5 will be described below.

The number of images B obtained by the intracorporeal and extracorporeal discrimination process of the image in S1 shown in FIG. 4 is assigned to a variable TB for indicating the number of total images used in the flow of FIG. 5. Variable CntB used as a counter is set at 1 (CntB = 1), and the first image data is read in the total pieces of image data B extracted in S1 (S20).

It is determined whether the image read in S20 is the first image (image of CntB = 1) or not (S21). Specifically, the flow goes to the direction of "Yes" if CntB = 1, and the flow goes to the direction of "No" if CntB ≥ 2. Because CntB = 1, the flow goes to the direction of "Yes", and a message that the image is "different" from the preceding image (a result message of "different") is returned (S25). Then, CntB is incremented (CntB = CntB + 1) .

Then, it is determined whether the processes are finished for the total pieces of image data B extracted in S1 or not. Specifically, the flow goes to the direction of "Yes" if TB < CntB, and the flow goes to the direction of "No" if TB ≥ CntB. In this case, because CntB = 2, the flow goes to the direction of "No" (if TB ≠ 1), the second image is read in S20, and the process of S21 is performed.

Because CntB = 2 in S21, the flow goes to the direction of "No", and the pixel values of the second image is compared to that of the preceding image (S22). At this point, for example, the difference in each pixel value between the current image and the corresponding preceding image may be computed. A sampling area may be previously determined to determine the difference in pixel value in the area. In this case, the speed enhancement of the process in S22 can be achieved compared with the case where the process in S22 is performed on the whole of the image.

The difference may be computed by computing the average pixel value of the whole of the current image and the average pixel value of the whole of the preceding image. The difference may be computed by computing the maximum (or minimum) pixel value in the pixels included in the current image and the maximum (or minimum) pixel value in the pixels included in the preceding image.

As a result of the comparison of two images in S22, when the difference between the pixel values of the two images (in other words, difference computed in S22) is not more than (or lower than) a predetermined threshold (S23), a message that the two images are the "identical image" (a result message of "identical image") is returned, and CntB is incremented (S24).

As a result of the comparison of two images in S22, when the difference between the pixel values of the two images is more than (or not lower than) the predetermined threshold (S23), the message that the two images are the "different image" (a result message of "different image") is returned, and CntB is incremented (S25).

When the processes are finished for the total pieces of image data B, because TB < CntB is satisfied in S26, the flow goes to the direction of "Yes", and the image determined as the "different image" in S25 is extracted (S27). Then, the flow is ended. In the first embodiment, the number of all images is used. However, even if the number of all images is not used, the process in which "the image files are sequentially read from the first image belonging to B file, and when the next file is found, the flow goes to the direction of 'No' in S26, otherwise the flow goes to the direction of 'Yes'." The number of images, which are determined as the "different image" and extracted in S27, is set at C.

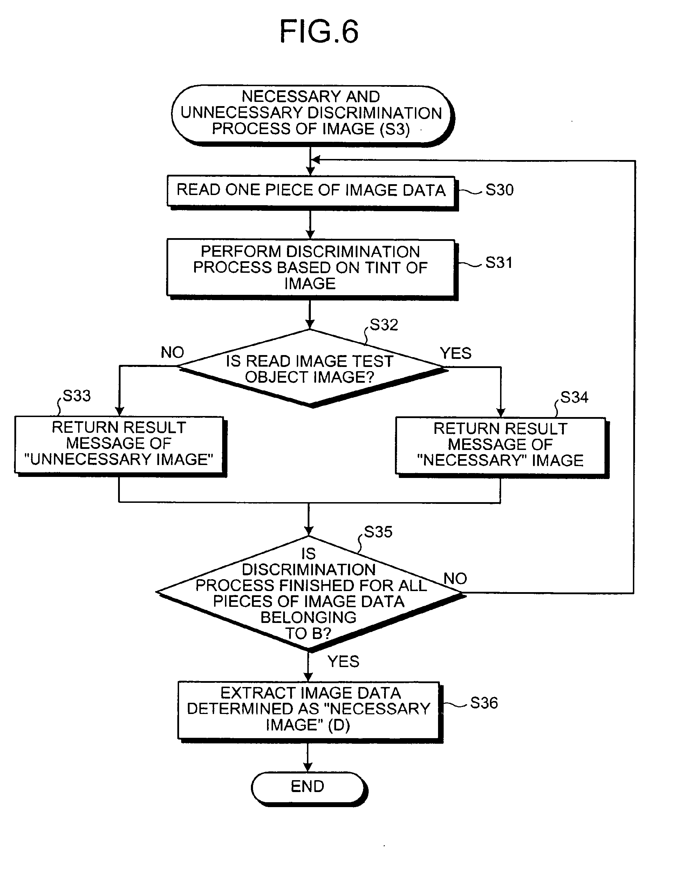

FIG. 6 is a view showing a detailed processing flow of the necessary and unnecessary discrimination process of the image in S3 of FIG. 3. In the flow of FIG. 6, only the image of the particular organ or region, i.e., only the necessary image is extracted from the images in which various organs or regions are taken. The flow of FIG. 6 will be described below.

The number of images C obtained by the intracorporeal and extracorporeal discrimination process of the image in S2 shown in FIG. 3 is assigned to a variable TC for indicating the number of total images used in the flow of FIG. 6. Variable CntC used as a counter is set at 1 (CntC = 1), and the first image data is read in the total pieces of image data C extracted in S2 (S30).

Then, a process of discriminating the tint of the image read in S30 is performed based on a predetermined threshold (S31). In the discrimination of S31, similarly to S13 of FIG. 4, the tint, i.e., the xy chromaticity value is determined, and it is determined whether the xy chromaticity value exists within the predetermined threshold range or not. S31 of FIG. 6 differs from S13 of FIG. 4 in threshold. The observation object region means a region to be diagnosed, i.e., an affection region. In the first embodiment, the extraction of the image in which a bleeding region is photographed will be described. The bleeding region is one of the affection regions.

The image which should be extracted in this flow is the image in which the observation object region is photographed, i.e., the image in which the bleeding region is photographed, so that it is necessary that the threshold be set such that the image in which the bleeding region is photographed is extracted. Therefore, the xy chromaticity value distribution of the bleeding region is previously computed, and the xy chromaticity value distribution is set at the threshold range.

Then, it is determined whether the image is the observation object image or not based on the result of S31 (S32). Specifically, a result message that the image is the "necessary image" is returned when the xy chromaticity value of the observation object image exists within the predetermined threshold range (S34), and a result message that the image is the "unnecessary image" is returned when the xy chromaticity value of the observation object image exists out of the predetermined threshold range (S34). Then, CntC is incremented (CntC = CntC + 1).

Then, it is determined whether the processes are finished for the total pieces of image data C to be processed in this flow or not (S35). Specifically, the flow goes to the direction of "Yes" if TC < CntC, and the flow goes to the direction of "No" if TC ≥ CntC. In this case, because CntA = 2, the flow goes to the direction of "No", the second image is read, the processes of S30 → S31 → S32 → S33 (or S34) → S35 are performed, and CntC is incremented. The same processes are performed on the images subsequent to the second image. The processes are repeated.

When the processes are finished for the total pieces of image data C, because TC < CntC is satisfied in S35, the flow goes to the direction of "Yes" in S35, and the image in which the result message of "necessary image" is returned in S34 is extracted (S36). Then, the flow is ended. In the first embodiment, the number of images extracted in S36 is set at D.

In the first embodiment, in FIG. 6, the tint is used to detect the image of the affection region (bleeding region in the above description). However, the invention is not limited to the tint. For example, the shapes of the affection regions such as an ulceration, a tumor, and an inflammation are previously registered, pattern matching is performed between the registered shape and the photographed image in S31, and the determination may be made by a degree of similarity.

In addition to the affection region, the image in which a predetermined organ is photographed can also be extracted. In this case, the value computed based on the tint of the organ which is of the observation object is used as the threshold. The organs of the body are different in tint, and each organ has the threshold based on the characteristic tint.

As described above, because D < C < B < A, the efficiency and shortening of the medical practice can be achieved by cutting down the number of images which should be watched in the diagnosis by the doctor. Thus, only the image of the affection region is extracted, and the images of other regions, the extracorporeal images, and substantially the same images can be removed. Therefore, an efficient medical practice and a shorter examination can be achieved by cutting down (extremely rapidly) the number of images which should be watched in the diagnosis by the doctor.

Second Embodiment
A second embodiment is a modification of the first embodiment, and a processing procedure is partially omitted and changed. The second embodiment will be described below.

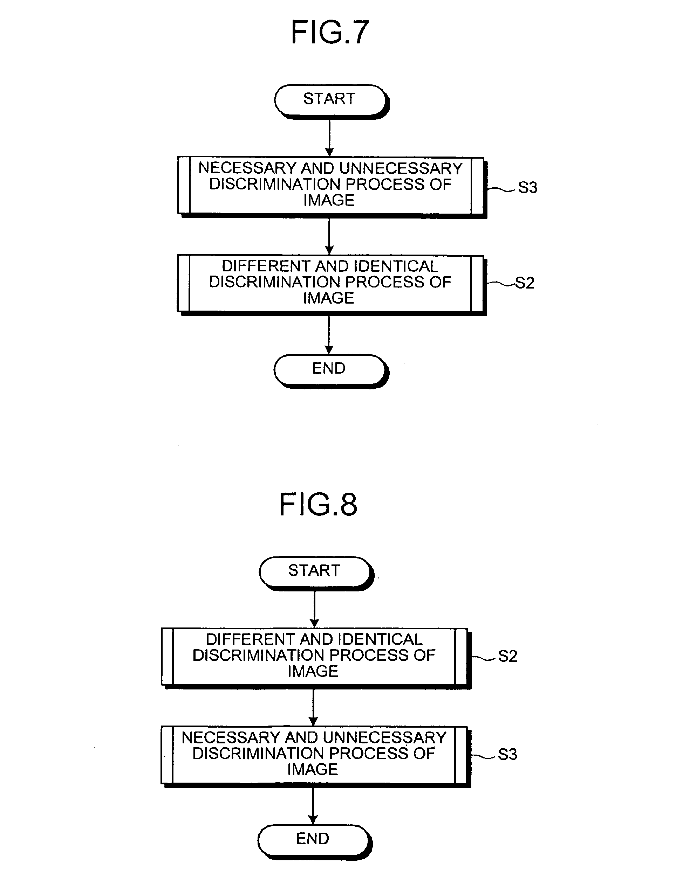

FIG. 7 is a view (example 1) showing a whole flow of image processing in the second embodiment. The flow of FIG. 7 differs from the flow of FIG. 3 in that S1 is neglected and the processing order of S2 and S3 is changed. Similarly to the flow described in FIG. 3 of the first embodiment, when the flow of FIG. 7 is performed, first the necessary and unnecessary discrimination process (S3) of the image is performed, and then the different and identical discrimination process (S2) of the image is performed.

When the processes are performed in the order of the flow shown in FIG. 7, the image of the affection region is extracted in S3, and then a group of images in which the same images are removed from the images of the affection region is extracted in S2.

The reason why the intracorporeal and extracorporeal discrimination process (S1) of the image of FIG. 3 is neglected is that the extracorporeal image is removed in FIG. 3. In other words, in the determination of S3 in which the tint is used, the threshold is set based on the tint of the bleeding portion of the bleeding region or the characteristic tint of the predetermined organ. Therefore, usually it is impossible that the tint of the extracorporeal image exceeds the threshold. However, there is no problem even if the processes are performed in the order of S1 → S3 → S2 without omitting S1.

When the threshold is not used but the pattern matching with the affection region is used, because the pattern of the affection region does not exist in the extracorporeal image, the extracorporeal image is never extracted in S3.

FIG. 8 is a view (example 2) showing a whole flow of the image processing in the second embodiment. The flow of FIG. 8 differs from the flow of FIG. 3 in that S1 is omitted. First the image in which the same images are removed from the images is extracted in S2, and then the image of the affection region is extracted in S3. As described above, the extracorporeal image is also removed in S3.

Thus, the same effect as the first embodiment is obtained in the second embodiment.

### INDUSTRIAL APPLICABILITY

An image processing apparatus, an image processing method, and an image processing program according to the present invention are suitable for image processing of an enormous amount of images taken by a medical instrument such as the capsule endoscope.

## Claims

1. An image processing apparatus which performs image processing of a plurality of images taken by a medical instrument, the image processing apparatus comprising:
an intracorporeal image determination unit that determines whether the image is an intracorporeal image obtained by photographing an inside of a body or not; and
an intracorporeal image extraction unit that extracts the intracorporeal image based on a determination result by the intracorporeal image determination unit.

2. The image processing apparatus according to claim 1, wherein the intracorporeal image determination unit determines whether the image is the intracorporeal image based on color component information on a pixel value included in the image.

3. The image processing apparatus according to claim 1, wherein the observation-object image determination unit compares a predetermined threshold to the color component information on the pixel value included in each image.

4. The image processing apparatus according to claim 3, wherein the threshold is based on the color component information on the pixel value of the observation object in the observation-object image.

5. The image processing apparatus according to claim 2, wherein the color component information is indicated by at least one of color components of tint elements x, y of an XYZ colorimetric system, tint elements u, v of a CIE U*V*W* color space, tint elements u', v' of a CIE LUV color space, tint elements a*, b* of a CIE LAB color space, and a ratio to an RGB signal value.

6. The image processing apparatus according to claim 1, wherein the intracorporeal image determination unit determines that all the undetermined images are the intracorporeal images when the intracorporeal image determination unit determines that a predetermined number of images among the plurality of images taken by the medical instrument are the intracorporeal images.

7. The image processing apparatus according to claim 1, further comprising:
an image identical determination unit that determines whether two given images are substantially identical to each other or different from each other among the intracorporeal images extracted by the intracorporeal image extraction unit; and
a different image extraction unit that extracts the different image based on a determination result by the image identical determination unit.

8. The image processing apparatus according to claim 7, wherein the image identical determination unit computes a difference between pixel values of two continuous intracorporeal images, and determines whether the two given images are substantially identical to each other or different from each other based on the difference.

9. The image processing apparatus according to claim 1, further comprising
an intracorporeal image determination unit that determines whether the image is an intracorporeal image obtained by photographing an inside of a body or not; and
an intracorporeal image extraction unit that extracts the intracorporeal image based on a determination result by the intracorporeal image determination unit.

10. The image processing apparatus according to claim 1, wherein the medical instrument is a capsule endoscope.

11. An image processing program which causes a computer to perform image processing of a plurality of images taken by a medical instrument, the image processing program causing the computer to perform:
an intracorporeal image determination step that determines whether the image is an intracorporeal image obtained by photographing an inside of a body or not; and
an intracorporeal image extraction step that extracts the intracorporeal image based on a determination result by the intracorporeal image determination step.

12. The image processing program according to claim 11, causing the computer to perform, in the intracorporeal image determination step, determining whether the image is the intracorporeal image based on color component information on a pixel value included in the image.

13. The image processing program according to claim 11, causing the computer to perform, in the observation-object image determination step, comparing a predetermined threshold to the color component information on the pixel value included in each image.

14. The image processing program according to claim 13, wherein the threshold is based on the color component information on the pixel value of the observation object in the observation-object image.

15. The image processing program according to claim 12, wherein the color component information is indicated by at least one of color components of tint elements x, y of an XYZ colorimetric system, tint elements u, v of a CIE U*V*W* color space, tint elements u', v' of a CIE LUV color space, tint elements a*, b* of a CIE LAB color space, and a ratio to an RGB signal value.

16. The image processing program according to claim 11, causing the computer to perform, in the intracorporeal image determination step, determining that all the undetermined images are the intracorporeal images when it is determined that a predetermined number of images among the plurality of images taken by the medical instrument are the intracorporeal images.

17. The image processing program according to claim 11, further causing the computer to perform:
an image identical determination step that determines whether two given images are substantially identical to each other or different from each other among the intracorporeal images extracted by the intracorporeal image extraction step; and
a different image extraction step that extracts the different image based on a determination result by the image identical determination step.

18. The image processing program according to claim 17, causing the computer to perform, in the image identical determination step,
computing a difference between pixel values of two continuous intracorporeal images; and
determining whether the two given images are substantially identical to each other or different from each other based on the difference.

19. The image processing program according to claim 11, further causing the computer to perform:
an intracorporeal image determination step that determines whether the image is an intracorporeal image obtained by photographing an inside of a body or not; and
an intracorporeal image extraction step that extracts the intracorporeal image based on a determination result by the intracorporeal image determination step.

20. The image processing program according to claim 11, wherein the medical instrument is a capsule endoscope.

21. An image processing method which performs image processing of a plurality of images taken by a medical instrument, the image processing method comprising:
an intracorporeal image determination step that determines whether the image is an intracorporeal image obtained by photographing an inside of a body or not; and
an intracorporeal image extraction step that extracts the intracorporeal image based on a determination result by the intracorporeal image determination step.

22. The image processing method according to claim 21, wherein the intracorporeal image determination step includes determining whether the image is the intracorporeal image based on color component information on a pixel value included in the image.

23. The image processing method according to claim 21, wherein the observation-object image determination step includes comparing a predetermined threshold to the color component information on the pixel value included in each image.

24. The image processing method according to claim 23, wherein the threshold is based on the color component information on the pixel value of the observation object in the observation-object image.

25. The image processing method according to claim 22, wherein the color component information is indicated by at least one of color components of tint elements x, y of an XYZ colorimetric system, tint elements u, v of a CIE U*V*W* color space, tint elements u', v' of a CIE LUV color space, tint elements a*, b* of a CIE LAB color space, and a ratio to an RGB signal value.

26. The image processing method according to claim 21, wherein the intracorporeal image determination step includes determining that all the undetermined images are the intracorporeal images when it is determined that a predetermined number of images among the plurality of images taken by the medical instrument are the intracorporeal images.

27. The image processing method according to claim 21, further comprising:
an image identical determination step that determines whether two given images are substantially identical to each other or different from each other among the intracorporeal images extracted by the intracorporeal image extraction step; and
a different image extraction step that extracts the different image based on a determination result by the image identical determination step.

28. The image processing method according to claim 27, wherein the image identical determination step includes computing a difference between pixel values of two continuous intracorporeal images, and determining whether the two given images are substantially identical to each other or different from each other based on the difference.

29. The image processing method according to claim 21, further comprising:
an intracorporeal image determination step that determines whether the image is an intracorporeal image obtained by photographing an inside of a body or not; and
an intracorporeal image extraction step that extracts the intracorporeal image based on a determination result by the intracorporeal image determination step.

30. The image processing method according to claim 21, wherein the medical instrument is a capsule endoscope.
